# EUROPEAN PATENT SPECIFICATION

(11) **EP 1 572 190 B1**
(45) Date of publication and mention of the grant of the patent: **18.04.2007**
(21) Application number: 03782419.0
(22) Date of filing: 16.12.2003
(51) Int. Cl.: A61K 31/216

(54) **FORMULATION COMPRISING FENOFIBRIC ACID, A PHYSIOLOGICALLY ACCEPTABLE SALT OR DERIVATIVE THEREOF**
FENOFIBRATSÄURE ENTHALTENDE PHARMAZEUTISCHE ZUSAMMENSETZUNG UND DEREN PHYSIOLOGISCH VERTRÄGLICHE SALZE UND DERIVATE
FORMULATION COMPORTANT DE L'ACIDE FENOFIBRIQUE, UN SEL OU UN DERIVE PHYSIOLOGIQUEMENT ACCEPTABLE DE CET ACIDE

(30) Priority: 17.12.2002 US 453694 P
(43) Date of publication of application: 14.09.2005
(73) Proprietor: Abbott GmbH & Co. KG, 65205 Wiesbaden (DE)
(72) Inventor: ROSENBERG, Jörg, 67158 Ellerstadt (DE); DEGENHARDT, Matthias, 67071 Ludwigshafen-Ruchheim (DE); BREITENBACH, Jörg, 68199 Mannheim (DE); REILAND, Tom, L., Surprise, Arizona 85387 (US); MARSH, Kennan, C., Lake Forest, 60046 (US)
(74) Representative: Wolter, Thomas
(86) International application number: PCT/EP2003/014331
(87) International publication number: WO 2004/054568

(56) References cited:
- EP-A- 0 173 293
- WO-A-01/03693
- WO-A-02/11699
- FR-A- 2 494 112
- US-A- 4 800 079
- US-A1- 2001 006 662

## Description

The present invention relates to formulations comprising fenofibric acid, a physiologically acceptable salt or derivative thereof, a process for their production, in particular by melt extrusion, and the use of these formulations for oral administration of fenofibric acid, a physiologically acceptable salt or derivative thereof.

Fenofibrate is a well-known lipid regulating agent which has been on the market for a long time.

Usually fenofibrate is orally administered. After its absorption which is known to take place in the duodenum and other parts of the gastrointestinal tract, fenofibrate is metabolized in the body to fenofibric acid. In fact, fenofibric acid represents the active principle of fenofibrate or, in other words, fenofibrate is a so-called prodrug which is converted in vivo to the active molecule, i.e. fenofibric acid. After oral administration of fenofibrate merely fenofibric acid is found in plasma.

Fenofibrate is known to be nearly insoluble in water requiring special pharmaceutical formulations to ensure good bioavailability, especially after oral administration. Accordingly, fenofibrate has been prepared in several different formulations, cf. WO 00/72825 and citations given therein, such as US-A 4,800,079, US-A 4,895,726, US-A 4,961,890, EP-A 0 793 958 and WO 82/01649. Further formulations of fenofibrate are described in WO 02/067901 and citations given therein, such as US-A 6,074,670 and US-A 6,042,847.

The products currently on the market are based on a formulation comprising micronized drug substance (TRICOR) in capsules and/or tablets. However, due to the insolubility of fenofibrate in water there is a tendency of said substance to recrystallize upon release from the formulation. This may reduce the bioavailability of the drug.

It is therefore an object of the present invention to provide formulations which make fenofibric acid sufficiently bioavailable and prevent recrystallization of fenofibric acid, physiologically acceptable salts or derivatives thereof prior to absorption.

This object is achieved by formulations which comprise fenofibric acid, a physiologically acceptable salt or a physiologically acceptable derivative thereof embedded in an enteric binder.

The present invention therefore relates to, preferably solid, formulations comprising
i) fenofibric acid, or a physiologically acceptable salt or derivative thereof and optionally other active substances;
ii) a binder component comprising at least one enteric binder; and optionally
iii) other physiologically acceptable excipients.

The term "formulation" means for the purposes of the present invention a mixture essentially composed of components i), ii) and optionally iii).

The term "fenofibric acid" refers according to the invention to 2-[4-(4-chlorobenzoyl)phenoxy]-2-methyl-propanoic acid, of the formula I

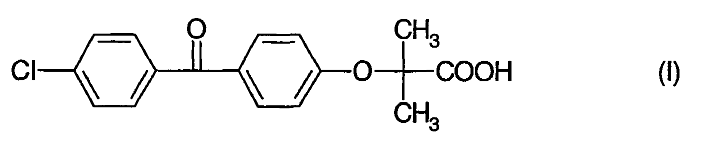

The physiologically acceptable salts in the present case are preferably base addition salts.

The base addition salts include salts with inorganic bases, for example metal hydroxides or carbonates of alkali metals, alkaline earth metals or transition metals, or with organic bases, for example ammonia, basic amino acids such as arginine and lysine, amines, e.g. methylamine, dimethylamine, trimethylamine, triethylamine, ethylamine, diethylamine, ethylenediamine, ethanolamine, diethanolamine, 1-amino-2-propanol, 3-amino-1-propanol or hexamethylenetetraamine, saturated cyclic amines having 4 to 6 ring carbon atoms, such as piperidine, piperazine, pyrrolidine and morpholine, and other organic bases, for example N-methylglucamine, kreatine and tromethamine, and quaternary ammonium compounds such as tetramethylammonium and the like. Preferred salts with organic bases are formed with amino acids. Preferred salts with inorganic bases are formed with Na, K, Mg and Ca cations.

The physiologically acceptable derivatives in the present case are preferably carboxylic acid derivatives which are reconvertable in vivo to the free carboxylic acid. Thus, preferred physiologically acceptable derivatives of fenofibric acid are prodrugs of fenofibric acid. The conversion of said prodrugs in vivo may occur under the physiological conditions which the prodrug experiences during its passage, or it may involve cleavage by enzymes, especially esterases, accepting said prodrug as substrate.

The physiological acceptable derivatives according to the present invention are in particular fenofibric acid derivatives of the formula II 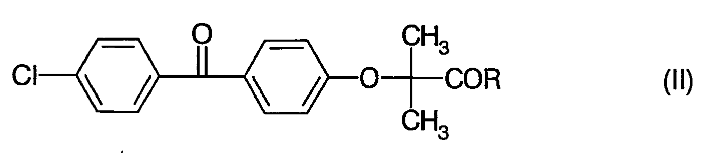 wherein R represents OR₁, -NR₁R₂, -NH-alkylene-NR₁R₂ or -O-alkylene-NR₁R₂, with R₁ and R₂ being identical or different from each other and representing a hydrogen atom, alkyl, alkoxyalkyl, alkoyloxyalkyl, alkoxycarbonyl, aminoalkyl, alkylaminoalkyl, dialkylaminoalkyl, trialkylammoniumalkyl, cycloalkyl, aryl or arylalkyl substituted on the aromatic residue by one or more halogen, methyl or CF₃ groups, or R₁ and R₂ forming - together with the nitrogen atom to which they are connected - a 5- to 7-membered aliphatic heterocyclic group which may enclose a second heteroatom selected from N, O, and S, and which may be substituted by one ore more halogen, methyl and/or CF₃ groups. Particularly preferred physiologically acceptable derivatives are fenofibric acid esters, i.e. derivatives of formula II wherein R represents OR₁ and R₁ is other than hydrogen. These esters in particular include derivatives of formula II wherein R₁ in -OR₁ represents an alkyl group having from 1 to 6 carbon atoms, an alkoxymethyl group having from 2 to 7 carbon atoms, a phenylalkyl group composed of an alkylene group having from 1 to 6 carbon atoms and a phenyl group, a phenyl group, an acetoxymethyl group, a pivaloyloxymethyl group, an ethoxycarbonyl group and a dimethylaminoethyl group.

Especially preferred according to the present invention are alkyl esters of fenofibric acid.

According to a particular embodiment, the present invention relates to formulations comprising i) the 1-methylethyl ester (isopropyl ester) of fenofibric acid, i.e. fenofibrate (INN).

The active substance component i) of the formulations of the invention comprises fenofibric acid, a physiologically acceptable salt or derivative thereof. Mixtures of these forms are possible, but will be considered only in certain cases. This part of the active substance component is for reasons of simplicity referred to hereinafter as fenofibric acid content.

Besides the fenofibric acid content, component i) of the formulations may comprise other active substances, in particular those with an action like that of fenofibric acid, e.g. other lipid regulating agents, such as further fibrates, e.g. bezafibrate, ciprofibrate and gemfibrocil, or statins, e.g. lovastatin, mevinolin, pravastatin, fluvastatin, atorvastatin, itavastatin, mevastatin, rosuvastatin, velostatin, synvinolin, simvastatin, cerivastatin and numerous others mentioned in, for instance, WO 02/067901 and the corresponding citations therein as well as expedient active substances of other types, which are incorporated herein by reference. One embodiment of the present invention comprises single-drug products which comprise an active substance component i) that essentially consists of fenofibric acid or a physiologically acceptable salt of fenofibric acid or a physiologically acceptable derivative of fenofibric acid or of a mixture thereof.

The active substance component and, in particular, the fenofibric acid content ordinarily constitutes 5 to 60% by weight, preferably 7 to 40% by weight and, in particular, 10 to 30% by weight of the formulation. Data in % by weight are based, unless indicated otherwise, on the total weight of the formulation.

The term "essentially" refers according to the invention usually to a percentage ratio of at least 90%, preferably of at least 95% and in particular of at least 98%.

The formulation base of formulations of the invention comprises physiologically acceptable excipients, namely at least one binder and optionally other physiologically acceptable excipients. Physiologically acceptable excipients are those known to be usable in the pharmaceutical technology sectors and adjacent areas, in particular those listed in relevant pharmacopeias (e.g. DAB, Ph. Eur., BP, NF, USP), as well as other auxiliary agents (excipients) whose properties do not impair a physiological use.

The binder component of the formulations of the invention may also be understood as binder which at least in part forms a binder matrix, in particular a polymer matrix, in which the active substance is embedded. Binders for the purpose of the invention are, in particular, solid meltable solvents. The binder matrix serves in particular to take up and, especially, to dissolve at least part of the active substance component, especially the fenofibric acid content. To this extent the binder is also, in particular, a solvent. In relation to active substance which is in the form of a molecular dispersion and dissolved, it is possible to speak of a solid solution of the active substance in the binder, the binder being either in crystalline form or, preferably, in amorphous form.

The binder component is preferably at least partly soluble or swellable in aqueous media, expediently under the conditions of use, that is to say in particular physiological conditions. An enteric binder may be defined as a binder, the solubility or swellability of which increases with increasing pH and vice versa. Particularly preferred are binders which are at least partly soluble or swellable in aqueous media having a pH of from 5 to 9, advantageously from 6 to 8 and more advantageously from 6,5 to 7,5.

Within the framework of the present description, aqueous media include water and mixtures of water and other components which comprise at least 50% by weight, preferably at least 70% by weight and in particular at least 90% by weight of water. Aqueous media include in particular body fluids such as fluids of the digestive tract, e.g. gastric juices, intestinal juices and saliva, blood; aqueous vehicles for use in pharmaceutical formulations in the drugs sector, e.g. vehicles which can be administered orally or parenterally, such as drinking water or water for injections.

Swelling refers to a process in which the volume and/or shape of a solid body, for example of a solid formulation of the invention, change on exposure to liquids, vapors and gases. Swellable or soluble are, in particular, hydrophilic polymers which are able to accumulate water at least on the surface and/or take up water between the polymer chains, mainly by absorption. Limited swelling usually results in gel formation, which is why polymers capable of limited swelling and usable according to the invention can be selected from the polymers commonly known as gel formers. Unlimited swelling usually leads to the formation of solutions or colloidal solutions, which is why polymers capable of unlimited swelling and usable according to the invention can be selected from the polymers which form at least colloidal solutions in the particular aqueous medium. It is expidient to take into account, especially in relation to body fluids, in particular those of the gastrointestinal tract, that there may be local variations in the physiological conditions, especially the pH. As it is preferred, according to the invention, that the active substance is taken up mainly in the duodenum, jejunum and/or ileum, it is advantageous for the binder to be swellable or soluble under the conditions prevailing in the duodenum, jejunum and/or ilium. In particular, it is advantageous for only slight or, preferably, essentially no swelling or dissolution to take place in the preceding sections of the gastrointestinal tract, especially in the stomach.

According to a preferred embodiment of the invention at least one binder of the binder component is a polymeric material, advantageously an enteric polymer. The term "enteric polymer" is a term of the art referring to a polymer which is preferentially soluble in the less acid environment of the intestine relative to the more acid environment of the stomach. Enteric polymers are pH sensitive. Typically the polymers are carboxylated and interact (swell) very little with water at low pH, whilst at high pH the polymers ionise causing swelling, or dissolving of the polymer. The binder component can therefore be designed to remain intact in the acidic environment of the stomach (protecting either the drug from this environment or the stomach from the drug), but to dissolve in the more alkaline environment of the intestine.

The enteric polymer may be an essentially conventional material. It is preferred for at least one binder of the binder component to be selected from enteric polymers such as suitable cellulose derivatives, e.g. cellulose acetate phthalates, cellulose acetate succinates, cellulose acetate trimellitates, carboxyalkyl(alkyl)celluloses and hydroxyalkyl(alkyl)cellulose phthalates; suitable polyvinyl-based polymers and copolymers, e.g. polyvinylacetatephthalate, polyvinylbutyrate acetate, vinyl acetate-maleic anhydride copolymer, styrene-maleic mono-ester copolymer; and suitable acrylic/methacrylic polymers and copolymers, e.g. alkyl acrylate-methacrylic acid copolymers such as methyl acrylate-methacrylic acid copolymers, and alkyl methacrylate-methacrylic acid-alkyl acrylate copolymers such as methacrylate-methacrylic acid-octyl acrylate copolymers.

Preferred enteric binders are the pharmaceutically acceptable acrylic/methacrylic acid polymers and copolymers. These include copolymers with anionic characteristics based on (meth)acrylic acid and alkyl (meth)acrylic acid esters such as methyl (meth)acrylate. These copolymers preferably have weight average molecular weights of around 50 000 to 300 000, in particular 100 000 to 150 000, e.g. around 135 000. The ratio of free carboxyl groups to esterified carboxyl groups of said copolymers is preferably in the range of around 2:1 to 1:3, in particular 1:1 to 1:2. Specific examples of said copolymers include the acrylic resins having the proprietary names Eudragit® L and S which are based on methacrylic acid and methyl methacrylate having a ratio of free carboxyl groups to esterified carboxyl groups of around 1:1 and 1:2, respectively. Among these, copolymers of the Eudragit® L type are preferred.

Particular preference is given to Eudragit® L 100, a pH dependent anionic polymer solubilizing above pH 6.0 for targeted drug delivery in the jejunum; and Eudragit® S 100, a pH dependent anionic polymer solubilizing above pH 7.0 for targeted drug delivery in the ileum.

Further preferred enteric binders are the pharmaceutically acceptable cellulose derivatives. These include carboxymethylethylcellulose (CMEC) and carboxymethylcellulose sodium (sodium cellulose glycolate), and more preferably hydroxypropylmethylcellulose phthalate, especially hypromellose phthalates such as 220824 and 220731, hydroxypropylmethylcellulose acetate succinate (AQOAT), cellulose acetate phthalate (CAP), and cellulose acetate trimellitate (CAT).

Such polymers are sold, for instance, under the trade name Cellacefate® (cellulose acetate phthalate) from Eastman Chemical Co., Aquateric® (cellulose acetate phthalate aqueous dispersion) from FMC Corp., Aqoat® (hydroxypropylmethylcellulose acetate succinate aqueous dispersion), and HP50 and HP55 (hydroxypropylmethylcellolose phthalates) from ShinEtsu K.K.

Further enteric binders include casein.

These enteric binders may be used either alone or in combination, and optionally together with other binders than those mentioned above.

Thus, the binder component of the formulations of the invention comprises at least one of the enteric binders described above and in particular at least one of the enteric polymers. It may comprise other binders of these types and/or of other types. The properties of the formulation of the invention can be altered by the nature of the chosen binder(s) or the admixture of different binders. In particular, it is possible in this way to control the release of active substance.

In one embodiment of the present invention, the binder component essentially consists of one of the enteric binders described above. In another embodiment of the present invention, the enteric binder component consists of a mixture of at least two of the enteric binders described above. According to these two embodiments, the enteric binder(s) constitute(s) 100 % by weight of the binder component (ii).

In a further embodiment of the present invention the binder component comprises in addition to one or more than one enteric binder at least one other (non-enteric) binder. According to this embodiment, the enteric binder preferably constitutes 5 to 95 % by weight, more preferably 10 to 70 % by weight and, in particular, 30 to 60 % by weight of the binder component (ii).

If at least one other (non-enteric) binder is present, it is preferred that said other (non-enteric) binder to be used in combination with the enteric binder is selected from:
synthetic polymers such as polyvinyllactams, in particular polyvinylpyrrolidone (PVP); copolymers of vinyllactams such as N-vinylpyrrolidone, N-vinylpiperidone and N-vinyl-ε-caprolactam, but especially N-vinylpyrrolidone, with (meth)acrylic acid and/or (meth)acrylic esters, such as long-chain (meth)acrylates, e.g. stearyl (meth)acrylate, dialkylaminoalkyl (meth)acrylates, which may be quaternized, and maleic anhydride, vinyl esters, especially vinyl acetate, vinylformamide, vinylsulfonic acid or quaternized vinylimidazole; copolymers of vinyl acetate and crotonic acid; partially hydrolyzed polyvinyl acetate; polyvinyl alcohol; (meth)acrylic resins such as poly(hydroxyalkyl (meth)acrylates), poly(meth)acrylate, acrylate copolymers; polyalkylene glycols such as polypropylene glycols and polyethylene glycols, preferably with molecular weights above 1 000, particularly preferably above 2 000 and very particularly preferably above 4 000 (e.g. polyethylene glycol 6 000); polyalkylene oxides such as polypropylene oxides and, in particular polyethylene oxides, preferably of high molecular weight, especially with weight average molecular weights of more than 100 000; polyacrylamides; polyvinylformamide (where appropriate partially or completely hydrolyzed);
modified natural polymers, e.g. modified starches and modified celluloses, such as cellulose esters and, preferably cellulose ethers, e.g. methylcellulose and ethylcellulose, hydroxyalkylcelluloses, in particular hydroxypropylcellulose, hydroxyalkylalkylcelluloses, in particular hydroxypropylmethylcellulose or hydroxypropylethylcellulose; starch degradation products, in particular starch saccharification products, such as maltodextrin;
natural or predominantly natural polymers such as gelatin, polyhydroxyalkanoates, e.g. polyhydroxybutyric acid and polylactic acid, polyamino acids, e.g. polylysine, polyasparagine, polydioxanes and polypeptides, and mannans, especially galactomannans; and
nonpolymeric binders such as polyols, for example those described in WO 98/22094 and EP 0 435 450, in particular sugar alcohols such as maltitol, mannitol, sorbitol, cellobiitol, lactitol, xylitol and erythritol, and isomalt (Palatinit).

Of those aforementioned, the polymeric binders, in particular the modified natural polymers, especially modified starches and cellulose ethers, and in particular the synthetic polymers, especially polyvinylpyrrolidone and copolymers of vinyllactams are preferred.

It is particularly preferred for at least one other binder of the binder component to be selected from polyvinylpyrrolidones, e.g. Kollidon® K25, N-vinylpyrrolidone/vinyl acetate copolymers, especially copovidone, e.g. Kollidon® VA 64, and cellulose derivatives such as low molecular weight hydroxypropylcellulose, e.g. Klucel®EF with weight average molecular weights of about 45 000 to about 70 000 or about 80 000, and hydroxypropylmethylcellulose, e.g. Methocel® E3, E5 and E7.

Binder components technically preferred for the process are those which are melt-processable.

Polymers which are advantageous for use as polymeric binder are those which have a K value (according to H. Fikentscher, Cellulose-Chemie 13 (1932), pp. 58-64 and 71-74) in the range between 10 and 100, in particular between 15 and 80.

In a preferred embodiment, the binder component (ii) has a glass transition temperature of more than 80°C, preferably of more than 90°C and in particular of more than 100°C. In addition, the suitability of glass transition temperatures in this range is governed by the necessary melt-processability of the binder or binder-containing mixtures.

The content of the binder component (ii) in the formulation of the invention is ordinarily from 20 to 95% by weight, preferably 30 to 90% by weight and in particular 40 to 85% by weight.

In a particular embodiment, the present invention relates to formulations wherein fenofibric acid, a physiologically acceptable salt or derivative thereof is in the form of a molecular dispersion.

The term "molecular dispersion" is known to the skilled artisan and describes essentially systems in which a substance, in the present case at least part and preferably the predominant part of the fenofibric acid content, is homogeneously dispersed in the binder component. In a molecular dispersion, the dispersed substance is free of interfaces. The binder in this case usually forms a matrix which, according to the invention, is formed by the binder component or at least by a predominant part of the binder component, advantageously the enteric binder.

According to this embodiment the content of active substance crystals in a formulation of the invention is preferably below 15% and in particular below 10%. Statements about crystal contents relate to the total amount of the active substance(s), in particular the fenofibric acid content.

A formulation of the invention which is essentially free of active substance crystals represents a particular embodiment of the present invention. The reduction in the crystal content is associated with an increase in the homogenization of the active substance in the matrix.

Molecular dispersion systems are, according to a particular embodiment, solid at room temperature (around 25 °C), but melt-processable at higher temperatures.

Formulations of the invention in which there are no crystalline contents for essentially any constituent (essentially amorphous or crystal-free formulations) represent a further particular embodiment of the present invention.

The state of such molecular dispersions can be investigated by known analytical methods, e.g. by differential scanning calorimetry (DSC) or wide-angle X-ray scattering measurements (WAXS measurements). Measurement of a molecular dispersion in DSC analysis lacks the, usually endothermic, melting peak occurring with the crystalline pure substance. Another possibility for identifying a molecular dispersion is the reduction in intensity and/or absence of typical X-ray diffraction signals in WAXS analysis.

For the purpose of forming molecular dispersions and, in particular, solid solutions by at least part of the active substance component in the binder component, the content of active substance component based on the binder component is advantageously from 1 to 50% by weight, preferably 10 to 40% by weight and in particular 20 to 30% by weight.

Formulations of the invention may, besides binder component, contain further physiologically acceptable excipients (excipient component iii). Such excipients may facilitate production of the formulation and/or modulate its properties. The nature and amount are advantageously chosen so that they do not impair development of the special properties of the formulations of the invention or contribute to destabilizing this system.

Excipients are usually conventional pharmaceutical excipients, for example,
fillers such as sugar alcohols, e.g. lactose, microcrystalline cellulose, mannitol, sorbitol and xylitol, isomalt (cf. DE 195 36 394), starch saccharification products, talc, sucrose, cereal corn or potato starch, where present in particular in a concentration of 0.02 to 50, preferably 0.20 to 20, % by weight based on the total weight of the mixture;
lubricants, glidants and mold release agents such as magnesium, aluminum and calcium stearates, talc and silicones, and animal or vegetable fats, especially in hydrogenated form and those which are solid at room temperature. These fats preferably have a melting point of 30°C or above. Technically preferred in relation to the melt extrusion process are - as described in DE 197 31 277 - triglycerides of C₁₂, C₁₄, C₁₆ and C₁₈ fatty acids or - to improve the processing properties - sodium stearylfumarate, lecithin, as described in connection with the extrusion of an isomalt-containing polymer/active substance melt in DE 195 36 394. It is also possible to use waxes such as carnauba wax. These fats and waxes may advantageously be admixed alone or together with mono- and/or diglycerides or phosphatides, in particular lecithin. The mono- and diglycerides are preferably derived from the abovementioned fatty acid types. Where present, the total amount of excipients in the form of lubricants and mold release agents is preferably 0.1 to 10% by weight and, in particular, 0.1 to 2 % by weight, based on the total weight of the mixture;
flow regulators, e.g. colloidal silica (highly dispersed silicon dioxide), especially the high-purity silicon dioxides having the proprietary name Aerosil®, where present in particular in an amount of 0.1 to 5% by weight based on the total weight of the mixture;
dyes such as azo dyes, organic or inorganic pigments or dyes of natural origin, with preference being given to inorganic pigments e.g. iron oxides, where present in a concentration of 0.001 to 10, preferably 0.1 to 3% by weight, based on the total weight of the mixture;
stabilizers such as antioxidants, light stabilizers, hydroperoxide destroyers, radical scavengers, stabilizers against microbial attack;
plasticizers, especially those described below.

It is also possible to add wetting agents, preservatives, disintegrants, adsorbents and mold release agents, and surfactants, especially anionic and nonionic, such as, for example, soaps and soap-like surfactants, alkyl sulfates and alkylsulfonates, salts of bile acids, alkoxylated fatty alcohols, alkoxylated alkylphenols, alkoxylated fatty acids and fatty acid glycerol esters, which may be alkoxylated, and solubilizers such as Cremophor® (polyethoxylated castor oil), Gelucire®, Labrafil® vitamin E TPGS and Tween® (ethoxylated sorbitan fatty acid esters) (cf., for example, H. Sucker et al. Pharmazeutische Technologie, Thieme-Verlag, Stuttgart 1978).

Excipients for the purpose of the invention also mean substances for producing a solid solution with the active substance. Examples of these excipients are pentaerythritol and pentaerythritol tetraacetate, urea, phosphatides such as lecithin, polymers such as, for example, polyethylene oxides and polypropylene oxides and their block copolymers (poloxamers) and citric and succinic acids, bile acids, stearins and others as indicated, for example, by J. L. Ford, Pharm. Acta Helv. 61, (1986), pp. 69-88.

Also regarded as pharmaceutical excipients are additions of acids and bases to control the solubility of an active substance (see, for example, K. Thoma et al., Pharm. Ind. 51, (1989), pp. 98-101).

Excipients in the sense of the invention are also vehicles specific for the dosage form, i.e. appropriate for a particular dosage form, in particular peroral and, especially, tablets and capsules, also low-melting or liquid excipients such as polyalkylene glycols of low molecular weight, in particular polyethylene glycol and/or polypropylene glycol with weight average molecular weights of less than 1 000, water or suitable aqueous systems.

It is also possible to add excipients such as masking flavors and odor-masking agents, in particular sweeteners and odorants.

Further particular embodiments concerning excipients are based on expert knowledge as described, for example, in Fiedler, H.B., Lexikon der Hilfsstoffe für Pharmazie, Kosmetik, und angrenzende Gebiete, 4th edition, Aulendorf: ECV-Editio-Cantor-Verlag (1996).

The only requirement for the suitability of excipients is usually the compatibility with the active substances and excipients used. The excipients ought advantageously not to impair the pH-sensivity of the formulation and the pH-sensitivity of the formulation and the formation of molecular dispersions.

The excipient component in solid formulations of the invention preferably comprises at least one of the excipients described above. It may comprise other excipients of these types and/or other types.

One embodiment of the present invention comprises formulations with excipient component iii). In this case, the content of the other physiologically acceptable excipients in the formulations of the invention can be up to 75% by weight, preferably up to 60% by weight and, in particular, up to 40% by weight.

A particular embodiment of the present invention comprises formulations which comprise
i) fenofibric acid or fenofibrate;
ii) at least one binder selected from enteric polymers; and
iii)optionally other physiologically acceptable excipients, in particular a flow regulator, e.g. highly disperse silica gel.

The formulations of the invention preferably contain less than 7% by weight and, in particular, less than 4% by weight of water. A particular embodiment is represented by less than 2 % by weight of water.

From the viewpoint of a formulation which can be administered orally, it is particularly preferred for at least part of the binder component to be designed such that the release of active substance at acidic pH is delayed.

The formulations of the invention have a solid consistency. The term "solid" has in this connection the meaning assigned in relevant pharmacopeias in connection with pharmaceutical preparations. In the wider sense, solid formulations of the invention also include those with a semisolid consistency, which may result in particular with high fenofibrate contents. By this are meant viscous or highly viscous formulations which can be molded at room temperature. The suitability of semisolid formulations for being expediently processed, according to the invention in particular by means of extrusion, is important.

Accordingly, formulations of the invention are mainly used in the physiological, in particular in the medical, sector for humans and animals. In this sense, the formulations are used as or in dosage forms, i.e. the formulations of the invention have expedient forms appropriate for physiological practise, if necessary together with other excipients.

Thus, the term "dosage form" refers to any dosage form for administration of active substances to an organism, preferably to mammals, in particular humans, agricultural or domestic animals.

Conventional dosage forms include, in particular, (in alphabetical sequence) capsules, granules, pellets, powders, suspensions, suppositories, tablets.

Granules consist of solid grains of formulations of the invention, each grain representing an agglomerate of powder particles. Granules having a mean corn size in the range of 0,1, (e.g. 0,12) to 2 mm, preferably 0,2 to 0,7 mm, are of advantage. Granules are preferably intended for oral use as dosage form. The user can be offered single-dose preparations, for example granules packed in a small bag (sachet), a paper bag or a small bottle, or multidose preparations which require appropriate measuring. However, in many cases, such granules do not represent the actual dosage form, but are intermediates in the manufacture of particular dosage forms, for example tablet granules to be compressed to tablets, capsule granules to be packed into hard gelatin capsules, or instant granules or granules for oral suspension to be put in water before intake.

As capsules, the formulations of the invention are usually packed into a hard shell composed of two pieces fitted together or a soft, one-piece, closed shell, which may vary in shape and size. It is likewise possible for formulations of the invention to be encased or enveloped or embedded in a matrix in suitable polymers, that is to say microcapsules and microspherules. Hard and soft capsules consist mainly of gelatin, while the latter have a suitable content of plasticizing substances such as glycerol or sorbitol. Hard gelatin capsules are used to receive preparations of the invention which have a solid consistency, for example granules, powder or pellets. Soft gelatin capsules are particularly suitable for formulations with a semisolid consistency and, if required, also viscous liquid consistency.

Pellets are granules of formulations of the invention in the particle size range from about 0.5 to 2 mm in diameter. Both with a narrow particle size distribution, preferably from 0.8 to 1.2 mm, and with an essentially round shape, are preferred.

In semisolid preparations, formulations of the invention are taken up in a suitable vehicle. Appropriate bases are known to the pharmaceutical technologist.

Suppositories are solid preparations for rectal, vaginal or urethral administration. In order to be appropriate for the administration route, formulations of the invention in these drug forms are usually taken up in suitable vehicles, for example in fats which melt at body temperature, such as hard fat, macrogols, i.e. polyethylene glycols with molecular weights of 1 000 to 3 000 in various proportions, glycerol gelatin and the like.

Tablets are solid preparations in particular for oral use. The meaning of oral within the framework of the present invention is, in particular, that of the term "peroral", i.e. tablets for absorption or action of the active substance in the gastrointestinal tract. Particular embodiments are coated tablets, layered tablets, laminated tablets, tablets with modified release of active substance, matrix tablets, effervescent tablets or chewable tablets. The formulations of the invention usually comprise at least a part of the necessary tablet excipients, such as binders, fillers, glidants and lubricants, and disintegrants. Tablets of formulations of the invention may also if necessary comprise other suitable excipients. Mention should be made in this connection of excipients which assist tableting, for example lubricants and glidants, for example those mentioned above, with preference for flow regulators such as silica and/or lubricants such as magnesium stearate in particular for facilitating compaction.

Coated tablets additionally comprise suitable coating materials, for example film coating agents with coating aids, especially those mentioned below. Coated tablets include, in particular, sugar-coated tablets and film-coated tablets.

Powders are finely dispersed solids of formulations of the invention with particle sizes usually of less than 1 mm. The above statements about granules apply correspondingly.

Preference is given according to the invention to capsules packed with granules, powders or pellets of formulations of the invention, instant granules and granules for oral suspension composed of formulations of the invention with addition of masking flavors, and, in particular, tablets and coated tablets.

The dosage forms of the invention are usually packed in a suitable form. Pushout (blister) packs made of plastic and/or metal for solid dosage forms are frequently used.

The present invention also relates to a process for producing a formulation of the invention by mixing (blending) components i), ii) and optionally iii) to form a plastic mixture. Thus, to form the plastic mixture, at least two measures are necessary, on the one hand the mixing (blending) of the components forming the mixture, and on the other hand the plastification thereof, i.e. the conversion thereof into the plastic state. These measures may take place for one or more components or portions of components successively, intermeshingly, alternately or in another way. Accordingly, it is possible in principle for the conversion into the plastic state to take place concurrently during a mixing process, or for the mixture first to be mixed and then to be converted into the plastic state. A plurality of plastic mixtures differing in composition may be formed during a process and are mixed together and/or with other components or portions of components. For example, a premix of a portion of the components, e.g. excipient component and/or binder component, can be formulated to form granules, and the granules can then be converted, with the addition of other components, e.g. the active substance component, into a plastic mixture whose composition may correspond to that of the formulation. It is also possible for all the components first to be combined and then either converted into the plastic state at the same time of the mixing or first mixed and then converted into the plastic state.

The formation of a plastic mixture can take place by melting or - with additional input of mechanical energy, e.g. by kneading, mixing or homogenizing - else below the melting point of the mixture. The plastic mixture is preferably formed at temperatures below 220°C. The formation of the plastic mixture usually does not take place by one or more components being converted into a paste or partially dissolved with liquids or solvents, but takes place mainly or exclusively by thermal or thermal/mechanical action on the component(s), i.e. by thermal plastification. The plastic mixture is preferably formed by extrusion, particularly preferably by melt extrusion. The plastification process steps can be carried out in a manner known per se, for example as described in EP-A-0 240 904, EP-A-0 337 256, EP-A-0358 108, WO 97/15290 and WO 97/15291. The contents of these publications and, in particular, the statements about melt extrusion present therein are incorporated herein by reference.

In principle, there are two possible ways by which solubilization of the active substance can be achieved during melt extrusion. On the one hand, the extrusion process is carried out at a temperature which is higher than the melting point of the active substance and high enough for plastification of the binder. In this case the molten active substance can be solubilized in the plastified binder by means of mixing and kneading which takes place during extrusion (method A). On the other hand, if the solubility of the active substance is good, a solubilization in the plastified binder can take place without the need to melt the active substance. This situation is comparable to the dissolution of water-soluble compounds (e.g. sugar) in water which is also possible without the need for prior melting the compound (method B). Fenofibrate is an active substance with a relatively low melting point (approximately 80 °C) and therefore a melting of the active substance can be expected during extrusion which is carried out normally at temperatures higher than 80 °C according to method A.

Fenobibric acid has a melting point of 184 °C (Arzneimittel-Forschung 26, 885-909 (1976), see page 887) which is much higher than the melting point of fenofibrate. Therefore solubilization of fenofibric acid in the binder(s) may take place according to method B. Moreover, method B could be advantageous even for processing fenofibrate in order to prevent any chemical degradation of fenofibrate at temperatures exceeding the melting point of fenofibrate.

In addition to the melt extrusion technology, there are other known technologies for embedding active substances in binders in moleculardispers form. The most common technique uses organic solvents where both the active substance(s) and the excipients (binders) are soluble. The solution of both compounds (active substances and binder(s)) are combined and then the solvent is removed completely. This process has a number of disadvantages because it requires the use of organig solvents causing a lot of problems during manufacturing. Although being possible, this is not the preferred process according to the present invention.

Especially in the case of low melting compounds like fenofibrate the active substance can be placed in a beaker and heated together with the binder while the whole mixture is stirred. This technique also does not use organic solvents but is based on a batch-process requiring much longer stirring and heating as in the case of a continuous process like melt extrusion. This means that the residence time of the drug at high temperature is much longer increasing the risk of possible degradation of both active substance(s) and binder(s). Furthermore this process normally requires low-viscosity melts which are obtained by using e.g. PEG. Althouth being possible, this is not the preferred process according to the present invention.

It should be possible to convert the binder component into a plastic state in the complete mixture of all the components in the range from 30 to 200°C, preferably 40 to 170°C. The glass transition temperature of the mixture should therefore be below 220°C, preferably below 180°C. If necessary, it is reduced by conventional, physiologically acceptable plasticizing excipients.

Examples of such plasticizers are:
organic, preferably involatile compounds, such as, for example, C₇-C₃₀-alkanols, ethylene glycol, propylene glycol, glycerol, trimethylolpropane, triethylene glycol, butandiols, pentanols such as pentaerythritol and hexanols, polyalkylene glycols, preferably having a molecular weight of from 200 to 1 000, such as, for example, polyethylene glycols (e.g. PEG 300, PEG 400), polypropylene glycols and polyethylene/propylene glycols, silicones, aromatic carboxylic esters (e.g. dialkyl phthalates, trimellitic esters, benzoic esters, terephthalic esters) or aliphatic dicarboxylic esters (e.g. dialkyl adipates, sebacic esters, azelaic esters, citric and tartaric esters, in particular triethylcitrate), fatty acid esters such as glycerol mono-, di- or triacetate or sodium diethyl sulfosuccinate. The concentration of plasticizer is, where present, generally 0.5 to 30, preferably 0.5 to 10, % by weight based on the total weight of polymer and plasticizer and from 0,1 to 40, especially from 0,5 to 20 and more specifically from 1 to 10 % by weight based on the total weight of the extruded formulation. They can be added during extrusion by pumping the liquid directly into the extruder. Alternatively they can be granulated with the one or all of the other solid components of the formulation prior to extrusion.

The amount of plasticizer advantageously does not exceed 30% by weight based on the total weight of polymer and plasticizer so that - in the area of solid forms - storage-stable formulations and dosage forms showing no cold flow are formed. Accordingly, it is preferred that the glass transition temperature of the final formulation is at least 40 °C, preferably at least 50 °C.

The process of the invention can advantageously be carried out at temperatures below 220°C and preferably below 180°C, but above room temperature (25°C), preferably above 40°C. A preferred temperature range for the extrusion of formulations of the invention is 80 to 180°C. The process is carried out in particular in a temperature range extending 40°C, preferably 30°C, and particularly preferably 20°C, upward or downward from the softening point of the mixture of the components.

In certain cases it may be advantageous to add components or portions of components as solution or suspension in a solvent. Particularly expedient ones are low molecular weight volatile solvents, e.g. water, C₁-C₆-monoalcohols and ethers thereof, esters of C₁-C₆-monoalkanols with C₁-C₆-carboxylic acids, alkanes. Another solvent which can be used is liquid CO₂. Water-soluble active substances can be employed as aqueous solution or, optionally, be taken up in an aqueous solution or dispersion of the binder component or a portion thereof. Corresponding statements apply to active substances which are soluble in one of the solvents mentioned, if the liquid form of the components used is based on an organic solvent. The components to be employed according to the invention may contain small amounts of solvent, e.g. because of hygroscopicity, trapped solvent or water of crystallization. The total solvent content of the plastic mixture is preferably less than 15%, in particular less than 10%, and particularly preferably less than 5%. The plastic mixture is preferably formed without the addition of a solvent, i.e. in particular by solvent-free melt extrusion.

The components, i.e. active substance and/or binder and, where appropriate, other excipients, can first be mixed and then be converted into the plastic state and homogenized. This can be done by operating the apparatuses such as stirred vessels, agitators, solids mixers etc. alternately. Sensitive active substances can then be mixed in (homogenized), preferably in "intensive mixers" in plastic phase with very small residence times. The active substance(s) may be employed as such, i.e. in particular in solid form, or as solution, suspension or dispersion.

The plastification, melting and/or mixing takes place in an apparatus usual for this purpose. Extruders or heatable containers with agitator, e.g. kneaders (like those of the type mentioned hereinafter) are particularly suitable.

It is also possible to use as mixing apparatus those apparatuses which are employed for mixing in plastics technology. Suitable apparatuses are described, for example, in "Mischen beim Herstellen und Verarbeiten von Kunststoffen", H. Pahl, VDI-Verlag, 1986. Particularly suitable mixing apparatuses are extruders and dynamic and static mixers, and stirred vessels, single-shaft stirrers with stripper mechanisms, especially paste mixers, multishaft stirrers, especially PDSM mixers, solids mixers and, preferably mixer/kneader reactors (e.g. ORP, CRP, AP, DTB from List or Reactotherm from Krauss-Maffei or Ko-Kneader from Buss), trough mixers or internal mixers or rotor/stator systems (e.g. Dispax from IKA).

The process steps of mixing and plastification, that is to say in particular the melting, can be carried out in the same apparatus or in two or more apparatuses operating separately from one another. The preparation of a premix can be carried out in one of the mixing apparatuses described above and normally used in particular for granulation. Such a premix can then be fed directly for example into an extruder, and then be extruded where appropriate with the addition of other components.

It is possible in the process of the invention to employ as extruders single screw machines, intermeshing screw machines or else multiscrew extruders, especially twin screw extruders which are particularly suited to produce solid dispersions of a drug dissolved or dispersed in a polymer (cf. EP 0 580 860 A), corotating or counter-rotating and, where appropriate, equipped with kneading disks. If it is necessary in the extrusion to evaporate a solvent, the extruders are generally equipped with an evaporating section. Examples of extruders which can be used are those of the ZSK series from Werner & Pfleiderer.

The mixing apparatus is charged continuously or batchwise, depending on its design, in a conventional way. Powdered components can be introduced in a free feed, e.g. via a weigh feeder. Plastic compositions can be fed in directly from an extruder or via a gear pump, which is particularly advantageous if the viscosities and pressures are high. Liquid media can be metered in by a suitable pump unit.

The mixture which has been obtained by mixing and converting the polymer component, the active substance component and, where appropriate, other excipients into the plastic state is pasty, of high viscosity or low viscosity (thermoplastic) and can therefore also be extruded. The glass transition temperature of the mixture is advantageously below the decomposition temperature of all the components present in the mixture.

The formulation of the invention is suitable as plastic mixture - where appropriate after cooling or solidification - in particular as extrudate, for all conventional processes for manufacturing conventional oral dosage forms, in particular drug forms.

The present invention also relates to a process for producing dosage forms based on formulations of the invention. Thus, where the formulation can be produced by the above process, and the formulation can be converted into the required dosage form where appropriate with the addition of other excipients. This can be done by using shaping process measures such as shaping the plastic mixture, in particular by extrusion or melt extrusion, and shaping the plastic mixture, in particular the extrudate - where appropriate after cooling or solidification - for example by granulation, grinding, compression, casting, injection molding, tableting under pressure, tableting under pressure with heat. It is also possible to convert a formulation into a desired dosage form by introducing it into suitable vehicles. It is thus also possible to process solid formulations into semisolid or liquid formulations through the addition of suitable vehicles.

A large number of, in particular, solid dosage forms can be manufactured in this way. For example, powders or granules can be produced by grinding or chopping the solidified or at least partly solidified plastic mixture, and can be either used directly for treatment or, where appropriate with addition of conventional excipients, further processed to the above dosage, in particular drug forms, especially to tablets.

Dosage forms are preferably shaped before solidification of the plastic mixture and result in a form which can be employed for treatment where appropriate after coating in a conventional way.

The shaping to the dosage form before solidification can take place in a variety of ways depending on the viscosity of the plastic mixture, for example by casting, injection molding, compression, or calendering. This is done by conveying the plastic mixture described above in the process according to the invention to one or more shaping steps. The conveying can take place by pressing, pumping, e.g. with gear pumps, or, preferably, with an extruder.

The plastic mixture is particularly preferably formed in one or more, preferably one, extruder and conveyed by the latter or a downstream extruder to the shaping steps. It has proved to be advantageous in many cases to extrude on a downward incline and/or where appropriate provide a guide channel for transporting the extrudate, in order to ensure safe transport and prevent rupture of the extrudate.

It may also be advantageous, depending on the number and compatibility of the active substances to be employed, to employ multilayer extrudates, for example coextrudates, as described in WO 96/19963, in the process of the invention.

Multilayer solid dosage forms can be produced in particular by coextrusion, in which case a plurality of mixtures of one or more of the components described above are conveyed together into an extrusion die so that the required layer structure results. Different binders are preferably used for different layers.

Multilayer dosage forms preferably comprise two or three layers. They may be in open or closed form, in particular as open or closed multilayer tablets.

If the shaping takes place by coextrusion, the mixtures from the individual extruders or other units are fed into a common coextrusion die and extruded. The shape of the coextrusion dies depends on the required dosage form. Examples of suitable dies are those with a flat orifice, called slit dies, and dies with an annular orifice cross section. The design of the die depends on the formulation base used and, in particular, the binder component and the desired dosage form.

The first shaping step advantageously takes place when the extrudate emerges from the extruder through suitably shaped dies, draw plates or other orifices, for example through a breaker plate, a circular die or a slit die. This usually results in a continuous extrudate, preferably with a constant cross section, for example in the form of a ribbon or of a strand, preferably with a circular, oval, rounded or flat and broad cross section.

Suitable downstream shaping steps for extrudates are, for example, cold cut, that is to say the cutting or chopping of the extrudate after at least partial solidification, hot cut, that is to say the cutting or chopping of the extrudate while still in the plastic form, or pinching off the still plastic extrudate in a nip device. It is possible with hot or cold cut to obtain, for example, granules (hot or cold granulation) or pellets. Hot granulation usually leads to dosage forms (pellets) with a diameter of from 0.5 to 3 mm, while cold granulation normally leads to cylindrical products with a length to diameter ratio of from 1 to 10 and a diameter of from 0.5 to 10 mm. It is possible in this way to produce monolayer but also, on use of coextrusion, open or closed multilayer dosage forms, for example oblong tablets, pastilles and pellets. The dosage forms can be provided with a coating by conventional methods in a downstream process step. Suitable materials for film coatings are the polymers mentioned as enteric binders. Further shaping steps may also follow, such as, for example, rounding off the pellets obtained by hot or cold cut using rounding-off devices as described in DE-A-196 29 753.

It is particularly preferred for all the shaping steps to be carried out on the still plastic mixture or still plastic extrudate. Besides hot cut, where appropriate with subsequent rounding off, a particularly suitable process is one in which the plastic mixture is shaped to the dosage form in a molding calender. This is done by conveying a still plastic mixture or a still plastic extrudate to a suitable molding calender. Suitable molding calenders usually have molding rolls and/or belts for the shaping, with at least one of the molding rolls and/or at least one of the belts having depressions to receive and shape the plastic mixture. It is preferred to use a molding calender with counter-rotating molding rolls, with at least one of the molding rolls having on its surface depressions to receive and shape the plastic mixture. Suitable molding calenders and devices containing molding rolls are generally disclosed for example in EP-A-0 240 904, EP-A-0 240 906 and WO 96/19962, and suitable belts and devices containing belts are generally disclosed for example in EP-A-0 358 105, which are expressly incorporated herein by reference.

The shaping of the still plastic mixture or still plastic extrudate preferably takes place at melt temperatures below 220°C, particularly preferably below 180°C and very particularly preferably below 150°C, such as, for example, in the temperature ranges necessary to form the plastic mixture or at lower temperatures. If the shaping takes place at lower temperatures, it advantageously takes place at from 5 to 70°C, preferably 10 to 50°C and particularly preferably 15 to 40°C below the highest temperature reached on formation of the plastic mixture, but preferably above the solidification temperature of the plastic mixture.

Preference is given to formulations and dosage forms obtainable by one of the processes described above.

Formulation of the invention, where appropriate as dosage form, and thus an effective amount of active substance, are administered to the individual to be treated, preferably a mammal, in particular a human, agricultural or domestic animal. Whether such a treatment is indicated and what form it is to take depends on the individual case and may be subject to medical assessment (diagnosis) which includes the signs, symptoms and/or dysfunctions which are present, the risks of developing certain signs, symptoms and/or dysfunctions, and other factors. The formulations of the invention are ordinarily administered together or alternately with other products in such a way that an individual to be treated receives a daily dose of about 50 mg to 250 mg fenofibrate on oral administration.

The formulations and dosage forms of the invention are mainly used in pharmacy, for example in the pharmaceutical sector as lipid regulating agents.

The term "alkyl, alkoxy etc." includes straight-chain or branched alkyl groups, such as methyl, ethyl, n-propyl, iso-propyl, n-butyl, sec-butyl, isobutyl, tert-butyl, n-pentyl or n-hexyl, preferably having - if not stated otherwise - 1 to 18, in particular 1 to 12 and particularly preferably 1 to 6, carbon atoms;

The term "cycloalkyl" includes mono- or bicyclic alkyl groups, such as cyclopropyl, cyclobutyl, cyclopentyl, cyclohexyl, etc., preferably having - if not stated otherwise - 3 to 9, in particular 3 to 7 and particularly preferably 5 or 6, carbon atoms.

"Aryl" is preferably naphthyl and in particular phenyl.

The "heterocyclic group" is in particular a 5- or 6-membered heterocyclic radical which can be aromatic or non-aromatic (aliphatic), mono- or bicyclic, and/or benzo-fused. The non-aromatic radicals include nitrogen-containing heterocyclic radicals, such as piperidinyl and piperazinyl. These also include heterocyclic radicals which contain two or more different heteroatoms, such as morpholinyl.

The present invention is now to be illustrated, but not restricted, by the present example.

### Example 1

Fenofibrate (120 g corresponding to 15 % w/w) and HP 55 S (hydropropylmethylcellulose phthalate, ShinEtsu, 672 g corresponding to 84 % w/w) and colloidal silica (Aerosil 200, 8 g corresponding to 1 % w/w) were blended for 4 minutes in a turbula blender. The powder mixture was then extruded in a twinscrew extruder (screw diameter 18 mm) with an feeding of 1.0 kg/h at a temperature of the melt at 165 °C. A clear, transparent melt rope with a thickness of approximately 1.0 cm was extruded. This material was directly formed into tablets (oblong-shaped) by calendering between two co-rotating rollers. By this process clear, transparent tablets of high hardness were obtained having a tablet weight of approximately 550 mg.

### Example 2

The tablets according to example 1 were milled in laboratory mill and the resulting powder was analyzed by DSC between 20 and 250 °C (Mettler Toledo DSC-820; 8.45 mg in a closed pan at 10 K/min). No endothermic melting peaks were observed, indicating that the fenofibrate was present in the polymer matrix in non-crystalline form.

### Example 3

The powder deriving from milling of the tablets according to example 2 was analzed by WAXS (wide angle x-ray scattering; Bruker AXS D-5005). There were no distinct peaks visible in the WAXS indicating that no crystalline fenofibrate was present in the formulation.

### Example 4

The tablets according to example 1 were analyzed with respect to possible drug degradation by HPLC according to the method described in Eur. pharm. for fenobibratum. The amount of the two known impurities according to USP were as follows: Impurity A = 0.067 %, Impurity B = 0.071 %. Although the extrusion was performed at a temperature far higher (165 °C) than the melting point of fenofibrate (approximately 80 °C) degradation took place to a very minor amount only.

### Example 5

Drug dissolution from the tablets according to example 1 was measured according to the USP paddle method at 37 °C in 900 ml aqueous solution of sodium dodecylsulfate (SDS, 0.05 mol/l) with a rotation speed of 75 rpm. Dissolution of the fenofibrate from the tablets was extremely slow in this medium. Only about 1 % of the fenofibrate was liberated even after 90 minutes.

### Example 6

The milled tablet material according to example 2 was screened (63 < x < 500 microns). Hard gelatine capsules (size 00, mean total capsule weight 740 mg) were filled with a powder mixture containing the sreened material (555 mg/capsule) together with mannitol (75 mg/capsule) and Aerosil 200 (5.55 mg/capsule). These capsules contained 83.25 mg fenofibrate.

### Example 7

Drug dissolution from the capsules according to example 6 was analyzed by the USP paddle method according to example 5 in 0.05 mol/l SDS solution. Fenofibrate release was shown to be faster compared to the unmilled tablets but was again relatively slow (16 % dissolution after 90 minutes).

### Example 8

Drug dissolution from the capsules according to example 6 was analyzed by the USP paddle method at 37 °C in 900 ml phosphate buffer (pH 6.8) additionally containing sodium dodecylsulfate (SDS, 0.05 mol/l) with a rotation speed of 75 rpm. At this pH the dissolution was significantly faster compared to the unbuffered aqueous medium (91 % dissolution after 90 minutes).

### Example 9

Dissolution analysis was performed according to example 8, but with a phosphate buffer having a pH of 7.2 together with 0.05 mol/l SDS. Drug dissolution was nearly 100 % after 90 minutes.

### Example 10

The capsules according to example 6 were tested with respect to bioavailability in the dog model (n = 4 dogs were used in this study, fasted). The marketed product (Tricor capsules, 67 mg fenofibrate/capsule) was used as reference. Plasma concentrations of fenofibric acid were determined by HPLC-MS. The results showed a remarkable increase in bioavailability for the formulation according to the present invention (approximately 4-fold increase in AUC) compared to the Tricor capsules.

### Example 11

Fenofibrate (150 g corresponding to 15 % w/w) and HP 50 (hydroxypropylmethylcellulose phthalate, ShinEtsu, 215 g corresponding to 21.5 % w/w) and PVP (Kollidon K25, BASF, 625 g corresponding to 62.5 % w/s) and colloidal silica (Aerosil 200, 10 g corresponding to 1 % w/w) were blended for 4 minutes in a turbula blender. The powder mixture was then extruded in a twin-screw extruder (screw diameter 18 mm) with a feeding of 1.4 kg/h at a temperature of the melt at 149 °C. A clear, transparent melt rope with a thickness of approximately 1.0 cm was extruded. This material was directly formed into tablets (oblong-shaped) by calendering between two co-rotating rollers. By this process opaque, translucent tablets of high hardness were obtained having a tablet weight of approximately 550 mg.

### Example 12

Fenofibrate (150 g corresponding to 15 % w/w) and HP 50 (hydroxpropylmethylcellulose phthalate, ShinEtsu, 190 g corresponding to 19 % w/w), PVP (Kollidon K25, BASF, 600 g corresponding to 60 % w/w) and polyoxyethylated oleic glyceride (Labrafil M 1944 CS, Gattefossee, 50 g corresponding to 5 % w/w) and colloidal silica (Aerosil 200, 10 g corresponding 1 % w/w) were blended for 4 minutes in a turbula blender. The liquid compound (Labrafil M 1944 CS) was granulated with the PVP prior to extrusion. The powder mixture including all ingredients was then extruded in a twin-screw extruder (screw diameter 18 mm) with a feeding of 2.0 kg/h at a temperature of the melt at 145 °C. A clear, transparent melt rope with a thickness of approximately 1.0 cm was extruded. This material was directly formed into tablets (oblong-shaped) by calendering between two co-rotating rollers. By this process opaque, translucent tablets of high hardness were obtained having a tablet weight of approximately 550 mg.

### Example 13

Fenobibric acid (120 g corresponding to 15 % w/w) and HP 55 (hydroxypropylmethylcellulose phthalate, ShinEtsu, 672 g corresponding to 85 % w/w) and colloidal silica (Aerosil 200, 8 g corresponding to 1 % w/w) were blended and extruded as outlined in example 1. A clear drug-containing melt was obtained. Transparent tablets with high hardness were obtained having a tablet weight of approximately 550 mg (corresponding to 82,5 mg fenofibric acid per tablet).

### Example 14

The crystallinity of the drug in the melt-extruded samples of example 13 were analyzed with respect to DSC and WAXS according to examples 2 and 3. No crystalline drug material was detected neither by DSC nor by WAXS.

### Example 15

Hard gelatine capsules were prepared according to example 6 containing milled extrudate (63< x < 500 microns) of the melt-extrudate of example 13. These capsules contained 73.79 mg fenofibric acid (mean) corresponding to 83.53 mg fenofibrate (f = 1.132), 413.23 mg HP 66 (mean), 134,63 mg mannitol (mean) and 11.57 mg Aerosil 200 (mean). The total weight of these caspules was 747.1 mg (mean).

### Example 16

The bioavailability of the capsule formulation according to example 15 (containing fenofibric acid) was tested with respect to bioavailability in the dog model in comparison to the capsule formulation according to example 6 (which contains fenofibrate). The bioavailability of the fenofibric acid-containing capsule (according to example 15) was shown to be twice as high as in the case of the fenofibrate-containing capsule formulation (according to example 6).

## Claims

1. A formulation comprising
i) fenofibric acid, or a physiologically acceptable salt or derivative thereof, and optionally other active substances;
ii) a binder component comprising at least one enteric binder; and optionally
iii) other physiologically acceptable excipients.

2. The formulation as claimed in claim 1, wherein the physiologically acceptable derivative of fenofibric acid is fenofibrate.

3. The formulation as claimed in claim 1, wherein fenofibric acid, the physiologically acceptable salt or derivative thereof is in the form of a molecular dispersion.

4. The formulation as claimed in claim 1, wherein the enteric binder is an enteric polymer.

5. The formulation as claimed in claim 4, wherein the enteric polymer is selected from hydroxypropylmethylcellulose phthalate, hydroxypropylmethylcellulose acetate succinate, carboxymethylethylcellulose, cellulose acetate phthalate, cellulose acetate trimellitate and carboxymethylcellulose sodium.

6. The formulation as claimed in claim 4, wherein the enteric polymer is selected from copolymers based on (meth)acrylic acid and at least one alkyl (meth)acrylic acid ester.

7. The formulation as claimed in claim 6, wherein the alkyl (meth)acrylic acid ester is methyl methacrylate.

8. The formulation as claimed in claim 6, wherein the copolymer has a ratio of free carboxyl groups to esterified carboxyl groups of around 2:1 to 1:3.

9. The formulation of claim 8, wherein the ratio is around 1:1.

10. The formulation as claimed in claim 1, wherein the formulation comprises
i) 5 to 60% by weight, preferably 7 to 40% by weight and in particular 10 to 30% by weight of active substance component;
ii) 20 to 95% by weight, preferably 30 to 90% by weight and in particular 40 to 85% by weight, of binder component;
iii)0 to 75% by weight, preferably 1 to 60% by weight and in particular 5 to 40% by weight, of other physiologically acceptable excipients.

11. The formulation as claimed in claim 1, wherein the enteric binder preferably constitutes 5 to 95 % by weight, more preferably 10 to 70 % by weight and, in particular, 30 to 60 % by weight of the binder component (ii).

12. The formulation as claimed in claim 1, wherein the content of active substance component (i) relative to binder component (ii) is from 1 to 50% by weight, preferably 10 to 40% by weight and in particular 20 to 30% by weight.

13. The formulation as claimed in claim 1, comprising
i) fenofibric acid or fenofibrate;
ii) at least one binder selected from enteric polymers; and optionally
iii)other physiologically acceptable excipients, especially a flow regulator, e.g. highly disperse silica gel.

14. The formulation as claimed in any one of the preceding claims, obtainable by melt extrusion of a mixture comprising fenofibric acid, a physiologically acceptable salt or derivative thereof, binder and optionally other active substances and/or other physiologically acceptable excipients.

15. Dosage form comprising a formulation as claimed in any one of claims 1 to 14.

## Patentansprüche

1. Formulierung, umfassend
i) Fenofibrinsäure oder ein physiologisch verträgliches Salz oder Derivat davon, und wahlweise weitere Aktivsubstanzen;
ii) eine Bindemittelkomponente, die wenigstens ein enterisches Bindemittel umfasst; und wahlweise
iii) weitere physiologisch verträgliche Exzipienten.

2. Formulierung nach Anspruch 1, wobei das physiologisch verträgliche Derivat der Fenofibrinsäure Fenofibrat ist.

3. Formulierung nach Anspruch 1, wobei Fenofibrinsäure, das physiologisch verträgliche Salz oder Derivat davon in Form einer molekularen Dispersion vorliegt.

4. Formulierung nach Anspruch 1, wobei das enterische Bindemittel ein enterisches Polymer ist.

5. Formulierung nach Anspruch 4, wobei das enterische Polymer ausgewählt ist unter Hydroxypropylmethylcellulosephthalat, Hydroxypropylmethylcelluloseacetatsuccinat, Carboxymethylethylcellulose, Celluloseacetatphthalat, Celluloseacetattrimellitat und Natriumcarboxymethylcellulose.

6. Formulierung nach Anspruch 4, wobei das enterische Polymer ausgewählt ist unter Copolymeren auf Basis von (Meth)acrylsäure und wenigstens einem Alkyl(meth)acrylsäureester.

7. Formulierung nach Anspruch 6, wobei der Alkyl(meth)acrylsäureester Methylmethacrylat ist.

8. Formulierung nach Anspruch 6, wobei das Copolymer ein Verhältnis von freien Carboxylgruppen zu veresterten Carboxylgruppen von etwa 2:1 bis 1:3 aufweist.

9. Formulierung nach Anspruch 8, wobei das Verhältnis etwa 1:1 beträgt.

10. Formulierung nach Anspruch 1, wobei die Formulierung
i) 5 bis 60 Gew.-%, vorzugsweise 7 bis 40 Gew.-% und insbesondere 10 bis 30 Gew.-% Aktivsubstanzkomponente;
ii) 20 bis 95 Gew.-%, vorzugsweise 30 bis 90 Gew.-% und insbesondere 40 bis 85 Gew.-% Bindemittelkomponente;
iii) 0 bis 75 Gew.-%, vorzugsweise 1 bis 60 Gew.-% und insbesondere 5 bis 40 Gew.-% weitere physiologisch verträgliche Exzipienten
umfasst.

11. Formulierung nach Anspruch 1, wobei das enterische Bindemittel vorzugsweise 5 bis 95 Gew.-%, bevorzugter 10 bis 70 Gew.-% und insbesondere 30 bis 60 Gew.-% der Bindemittelkomponente (ii) ausmacht.

12. Formulierung nach Anspruch 1, wobei der Gehalt an Aktivsubstanzkomponente (i) bezogen auf die Bindemittelkomponente (ii) 1 bis 50 Gew.-%, vorzugsweise 10 bis 40 Gew.-% und insbesondere 20 bis 30 Gew.-% berägt.

13. Formulierung nach Anspruch 1, umfassend
i) Fenofibrinsäure oder Fenofibrat;
ii) wenigstens ein unter enterischen Polymeren ausgewähltes Bindemittel; und wahlweise
iii) weitere physiologisch verträgliche Exzipienten, insbesondere ein Fließregulierungsmittel, beispielsweise hochdisperses Silicagel.

14. Formulierung nach einem der vorhergehenden Ansprüche, erhältlich durch Schmelzextrusion eines Gemisches, das Fenofibrinsäure, ein physiologisch akzeptables Salz oder Derivat davon, Bindemittel und wahlweise weitere Aktivsubstanzen und/oder weitere physiologisch verträgliche Exzipienten umfasst.

15. Dosierungsform, die eine Formulierung nach einem der Ansprüche 1 bis 14 umfasst.

## Revendications

1. Formulation comprenant
i) de l'acide fénofibrique, ou un sel ou dérivé physiologiquement acceptable de celui-ci, et facultativement d'autres substances actives ;
ii) un composant liant comprenant au moins un liant entérique ; et facultativement
iii) d'autres excipients physiologiquement acceptables.

2. Formulation selon la revendication 1, dans laquelle le dérivé physiologiquement acceptable de l'acide fénofibrique est le fénofibrate.

3. Formulation selon la revendication 1, dans laquelle l'acide fénofibrique, le sel ou dérivé physiologiquement acceptable de celui-ci est sous la forme d'une dispersion moléculaire.

4. Formulation selon la revendication 1, dans laquelle le liant entérique est un polymère entérique.

5. Formulation selon la revendication 4, dans laquelle le polymère entérique est choisi parmi le phtalate d'hydroxypropylméthylcellulose, l'acétate succinate d'hydroxypropylméthylcellulose, la carboxyméthyléthylcellulose, l'acétate phtalate de cellulose, l'acétate trimellitate de cellulose et la carboxyméthylcellulose sodique.

6. Formulation selon la revendication 4, dans laquelle le polymère entérique est choisi parmi des copolymères à base d'acide (méth)acrylique et au moins d'un ester d'alkyle d'acide (méth)acrylique.

7. Formulation selon la revendication 6, dans laquelle l'ester d'alkyle d'acide méthacrylique est le méthacrylate de méthyle.

8. Formulation selon la revendication 6, dans laquelle le copolymère a un rapport des groupes carboxyle libres sur les groupes carboxyle estérifiés d'environ 2 : 1 à 1 : 3.

9. Formulation selon la revendication 8, dans laquelle le rapport est d'environ 1:1.

10. Formulation selon la revendication 1, dans laquelle la formulation comprend
i) 5 à 60 % en poids, de préférence 7 à 40 % en poids et en particulier 10 à 30 % en poids, d'un composant de substance active ;
ii) 20 à 95 % en poids, de préférence 30 à 90 % en poids et en particulier 40 à 85 % en poids, d'un composant liant ;
iii) 0 à 75 % en poids, de préférence 1 à 60 % en poids et en particulier 5 à 40 % en poids, d'autres excipients physiologiquement acceptables.

11. Formulation selon la revendication 1, dans laquelle le liant entérique constitue de préférence 5 à 95 % en poids, de manière davantage préférée 10 à 70 % en poids et, en particulier, 30 à 60 % en poids du composant liant (ii).

12. Formulation selon la revendication 1, dans laquelle la teneur en le composant de substance active (i) par rapport au composant liant (ii) est de 1 à 50 % en poids, de préférence de 10 à 40 % en poids et en particulier de 20 à 30 % en poids.

13. Formulation selon la revendication 1, comprenant
i) de l'acide fénofibrique ou du fénofibrate ;
ii) au moins un liant choisi parmi des polymères entériques ; et facultativement
iii) d'autres excipients physiologiquement acceptables, en particulier un régulateur d'écoulement, par exemple un gel de silice hautement dispersé.

14. Formulation selon l'une quelconque des revendications précédentes, pouvant être obtenue par extrusion en fusion d'un mélange comprenant de l'acide fénofibrique, un sel ou dérivé physiologiquement acceptable de celui-ci, un liant et facultativement d'autres substances actives et/ou d'autres excipients physiologiquement acceptables.

15. Forme galénique comprenant une formulation selon l'une quelconque des revendications 1 à 14.
